# EUROPEAN PATENT APPLICATION

(11) **EP 2 397 556 A1**
(43) Date of publication of application: **21.12.2011**
(21) Application number: 11177420.4
(22) Date of filing: 01.05.2007
(51) Int. Cl.: C12P 7/06

(54) **Thermophilic organisms for conversion of lignocellulosic biomass to ethanol**

(30) Priority: 01.05.2006 US 796380 P; 31.10.2006 WO PCT/US2006/042442
(62) Divisional of application: 07783037.0
(71) Applicant: Trustees of Dartmouth College, Hanover, NH 03755-1404 (US); Mascoma Corporation, Cambridge MA 02142 (US)
(72) Inventor: Shaw, Arthur Josephus, IV, West Lebanon, NH 03766 (US); Desai, Sunil G., Ellicott City, MD 21042 (US); Lynd, Lee R., Meriden, NH 03770 (US); Tyurin, Mikhail V., Bain Bridge, GA 39819 (US); Podkaminer, Kara, Hanover, NH 03755 (US); Bardsley, John, Newport, NH 03773-1969 (US); Hogsett, David Anthony, Grantham, NH 03753 (US)
(74) Representative: Kudlek & Grunert Patentanwälte

(57) **Abstract**

Mutant thermophilic organisms that consume a variety of biomass derived substrates are disclosed herein. Strains of *Thermoanaerobacterium saccharolyticum* with acetate kinase and phosphotransacetylase expression eliminated are disclosed herein. Further, strain ALK1 has been engineered by site directed homologous recombination to knockout both acetic acid and lactic acid production. Continuous culture involving a substrate concentration challenge lead to evolution of ALK1, and formation of a more robust strain designated ALK2. The organisms may be utilized for example in thermophilic SSF and SSCF reactions performed at temperatures that are optimal for cellulase activity to produce near theoretical ethanol yields without expressing pyruvate decarboxylase.

## Description

### RELATED APPLICATIONS

This application claims priority to PCT/US06/042442, filed October 31, 2006, which claimed priority to U.S. Application No. 60/796,380, filed May 1, 2006. Each of these applications is incorporated herein by reference.

### GOVERNMENT INTERESTS

The United States Government may have certain rights in the present invention as research relevant to its development was funded by National Institute of Standards and Technology (NIST) contract number 60NANB1D0064.

### BACKGROUND

### 1. FIELD OF THE INVENTION

The present invention pertains to the field of biomass processing to produce ethanol. In particular, novel thermophilic organisms that consume a variety of biomass derived substrates and produce ethanol in high yield are disclosed, as well as processes for the production and use of the organisms.

### 2. Description of the Related Art

Biomass represents an inexpensive and readily available cellulolytic substrate from which sugars may be produced. These sugars may be used alone or fermented to produce alcohols and other products. Among bioconversion products, interest in ethanol is high because it may be used as a renewable domestic fuel.

Significant research has been performed in the areas of reactor design, pretreatment protocols and separation technologies, so that bioconversion processes are becoming economically competitive with petroleum fuel technologies. However, it is estimated that the largest cost savings may be obtained when two or more process steps are combined. For example, simultaneous saccharification and fermentation (SSF) and simultaneous saccharification and co-fermentation (SSCF) processes combine an enzymatic saccharification step with fermentation in a single reactor or continuous process apparatus. In an SSF process, end-product inhibition is removed as the soluble sugars are continually fermented into ethanol. When multiple organisms are used to provide a variety of hydrolysis products, the SSF process is usually referred to as a simultaneous saccharification and co-fermentation (SSCF) process. In addition to savings associated with shorter reaction times and reduced capital costs, co-fermentation processes may also provide improved product yields because certain compounds that would otherwise accrue at levels that inhibit metabolysis or hydrolysis are consumed by the co-fermenting organisms. In one such example, β-glucosidase ceases to hydrolyze cellobiose in the presence of glucose and, in turn, the build-up of cellobiose impedes cellulose degradation. An SSCF process involving co-fermentation of cellulose and hemicellulose hydrolysis products may alleviate this problem by converting glucose into one or mare products that do not inhibit the hydrolytic activity of β-glucosidase.

Consolidated bioprocessing (CBP) involves four biologically-mediated events: (1) enzyme production, (2) substrate hydrolysis, (3) hexose fermentation and (4) pentose fermentation. These events may be performed in a single step. This strategy requires a microorganism that utilizes cellulose and hemicellulose. Development of CBP organisms could potentially result in very large cost reductions as compared to the more conventional approach of producing saccharolytic enzymes in a dedicated process step. CBP processes that utilize more than one organism to accomplish the four biologically-mediated events are referred to as consolidated bioprocessing co-culture fermentations.

Some bacteria have the ability to convert pentose sugars into hexose sugars, and to ferment the hexose sugars into a mixture of organic acids and other products by glycolysis. The glycolytic pathway begins with conversion of a six-carbon glucose molecule into two three-carbon molecules of pyruvate. Pyruvate may then be converted to lactate by the action of lactate dehydrogenase ("*ldh*"), or to acetyl coenzyme A ("acetyl-CoA") by the action of pyruvate dehydrogenase or pyruvate-ferredoxin oxidoreductase. Acetyl-CoA is further converted to acetate by phosphotransacetylase and acetate kinase, or reduced to ethanol by acetaldehyde dehydrogenase ("AcDH") and alcohol dehydrogenase *("adh").* Overall, the performance of ethanol-producing organisms is compromised by production of organic products other than ethanol, and particularly by *ldh*-mediated conversion of pyruvate to lactate, and by conversion of acetyl-CoA to acetate by phosphotransacetylase and acetate kinase.

Metabolic engineering of bacteria has recently resulted in the creation of a knockout of lactate dehydrogenase in the thermophilic, anaerobic, Gram-positive bacterium *T. saccharolyticum.* See, Desai, S.G.; Guerinot, M.L.; Lynd, L.R. "Cloning of L-lactate dehydrogenase and elimination of lactic acid production via gene knockout in Thermoanaerobacterium saccharolyticum JW/SL-YS485" Appl. Microbiol. Biotechnol. 65: 600-605, 2004.

Although the knockout of *ldh* constitutes an advance in the art, it is problematic for some uses of this organism in that this strain of *T. saccharolyticum* continues to make organic acid - in particular, acetic acid.

### SUMMARY

The present instumentalities advance the art and overcome the problems outlined above by providing thermophilic, anaerobic bacteria that consume a variety of biomass derived substrates and produce ethanol in near theoretical yields. Methods for producing ethanol using the organisms are also disclosed.

The instrumentalities reported herein result in the knockout of various genes either singly or in combination, where such genes in the native organism would otherwise result in the formation of organic acids. For example, there may be knockouts of: (a) acetate kinase and/or phosphotransacetylase and (b) lactate dehydrogenase (ldh), acetate kinase (ack) and phosphotransacetylase *(pta)* in *T. saccharolyticum* JW/SL-YS485. Although the results reported herein are for *T*. *saccharolyticum,* the methods and materials also apply to other members of the *Thermoanaerobacter* genus including *Thermoanaerobacterium thermosulfurigenes, Thermoanaerobacterium aotearoense, Thermoanaerobacterium polysaccharolyticum, Thermoanaerobacterium zeae, Thermoanaerobacterium thermosaccharolyticum,* and *Thermoanaerobacterium xylanolyticum.* The methods and materials are useful generally in the field of metabolically engineered, thermophilic, Gram-positive bacteria.

In one embodiment, an isolated organism, which does not express pyruvate decarboxylase, ferments a cellulolytic substrate to produce ethanol in a concentration that is at least 90% of a theoretical yield.

In one embodiment, a Gram-positive bacterium, that in a native state contains at least one gene which confers upon the Gram-positive bacterium an ability to produce acetic acid as a fermentation product, is transformed to eliminate expression of the at least one gene. The bacterium may be a *thermoanaerobacter,* such as *Thermoanaerobacterium saccharolyticum.* The gene which confers upon the Gram-positive bacterium an ability to produce acetic acid as a fermentation product may code for expression of acetate kinase and/or phosphotransacetylase.

In another embodiment, the Gram-positive bacterium may be further transformed to eliminate expression of one or more genes that confer upon the Gram-positive bacterium the ability to produce lactic acid as a fermentation product. For example, the gene that confers the ability to produce lactic acid may be lactate dehydrogenase.

In one embodiment, a method for producing ethanol includes transforming a native organism to produce a Gram-positive bacterium that has been transformed to eliminate expression of all genes that confer upon the Gram-positive bacterium the ability to produce organic acids as fermentation products, to produce a transformed bacterial host, and culturing the transformed bacterial host in medium that contains a substrate including a material selected from the group consisting of glucose, xylose, cellobiose, sucrose, xylan, starch, and combinations thereof under suitable conditions for a period of time sufficient to allow saccharification and fermentation of the substrate.

In one embodiment, a biologically pure culture of a microorganism designated ALK1 and deposited with the ATCC under Patent Deposit Designation No. PTA-7206 is described.

In one embodiment an isolated polynucleotide comprises (a) a sequence of SEQ ID NO: 10, or (b) a sequence of SEQ ID NO: 9 and SEQ ID NO: 10, or (c) a sequence having at least about 90% sequence identity with the sequence of (a) or (b). A vector comprising the isolated polynucleotide of (a), (b), or (c) is described, as well as a host cell genetically engineered to express a compliment of the polynucleotide of (a), (b), or (c). In another embodiment, an isolated polynucleotide comprises a sequence having at least about 95% sequence identity with the sequence of (a) or (b). In yet another embodiment, an isolated polynucleotide comprises a sequence having at least about 98%, or at least about 99%, sequence identity with the sequence of (a) or (b).

In one embodiment, a method of producing ethanol includes culturing a mutant bacterium expressing a compliment of the isolated polynucleotide of (a), (b), or (c) in medium containing a substrate selected from the group consisting of glucose, xylose, cellobiose, sucrose, xylan, starch, and combinations thereof under suitable conditions for a period of time sufficient to allow fermentation of the substrate to ethanol.

In one embodiment, a method for producing ethanol includes providing within a reaction vessel, a reaction mixture comprising lignocellulosic substrate, cellulase and a fermentation agent. The fermentation agent comprises a Gram-positive bacterium that has been transformed to eliminate expression of at least one gene that confers upon the Gram-positive bacterium, in a native state, an ability to produce acetic acid as a fermentation product. The reaction mixture is reacted under suitable conditions for a period of time sufficient to allow saccharification and fermentation of the lignocellulosic substrate.

### BRIEF DESCRIPTION OF THE DRAWINGS

Fig. 1 shows reactions of the glycolytic pathway.

Fig. 2 shows hydrogen production in wild-type *T. saccharolyticum* compared to various knockout strains of *T. saccharolyticum.*

Fig. 3 shows a comparison of experimental and expected polynucleotide sequences for the *ldh* region of the suicide vector pSGD9 (SEQ ID NO: 9) integrated into the genome of *T saccharolyticum.*

Fig. 4 shows a comparison of experimental and expected polynucleotide sequences for the *pta*/*ack* region of the suicide vector pSGD8-Erm (SEQ ID NO: 10) integrated into the genome of *T. saccharolyticum.*

Figs. 5-7 show high-performance liquid chromotegraphy (UPLC) traces of a fermentation broth at various time intervals during growth of ALK1.

Fig. 8 shows xylose, organic acid and ethanol concentrations during fermentation by strain ALK1.

Fig. 9 shows xylose, organic acid and ethanol concentrations during fermentation by wild-type *T*. *saccharolyticum.*

Fig. 10 shows xylose, organic acid and ethanol concentrations during a continuous culture challenge of ALK1.

Fig. 11 shows xylose, organic acid and ethanol concentrations during fermentation by strain ALK2.

Fig. 12 shows ethanol production at various Avicel concentrations during thermophilic SSF reactions involving ALK2 and cellulase at 50°C.

Fig. 13 shows ethanol yield for the thermophilic SSF reactions shown in Fig.12.

### DETAILED DESCRIPTION

There will now be shown and described methods for engineering and utilizing thermophilic, anaerobic, Gram-positive bacteria in the conversion of biomass to ethanol.

As used herein, an organism is in "a native state" if it is has not been genetically engineered or otherwise manipulated by the hand of man in a manner that intentionally alters the genetic and/or phenotypic constitution of the organism. For example, wild-type organisms may be considered to be in a native state.

Complete elimination of organic acid production from a *T*. *saccharolyticum* in a native state was achieved using two site-directed DNA homologous recombination events. The mutant strain, *Thermoanaerobacterium saccharolyticum* JW/SL-YS485 ALK1 ("ALK1") produces near theoretical amounts of ethanol at low substrate feedings in batch culture with a temperature in a range of about 30-66°C and a pH in a range of about 3.85-6.5. In one embodiment, ethanol yield is at least about 90% of the theoretical maximum. ALK1, and its decendents, have the potential to contribute significant savings in the lignocellulosic biomass to ethanol conversion due to its growth conditions, which are substantially optimal for cellulase activity in SSF and SSCF processes. For example, optimal cellulase activity parameters include a pH between 4-5 and temperature between 40-50°C. Additionally, it is unnecessary to adjust the pH of the fermentation broth when knockout organisms, which lack the ability to produce organic acids, are used. ALK1, and similar organisms, may also be suitable for a consolidated bioprocessing co-culture fermentation where the knockout organism would convert pentoses to ethanol, and cellulose would be degraded by a cellulolytic organism such as C. *thermocellum.*

Operating either an SSF, SSCF or CBP process at thermophilic temperatures offers several important benefits over conventional mesophilic fermentation temperatures of 30-37°C. In particular, enzyme concentrations necessary to achieve a given amount of conversion may be reduced due to higher enzyme activity at thermophilic temperatures. As a result, costs for a process step dedicated to cellulase production are substantially reduced (e.g., 2-fold or more) for thermophilic SSF and SSCF, and are eliminated for CBP. Costs associated with fermentor cooling and also heat exchange before and after fermentation are also expected to be reduced for thermophilic SSF, SSCF and CBP. Finally, processes featuring thermophilic biocatalysts may be less susceptible to microbial contamination as compared to processes featuring conventional mesophilic biocatalysts.

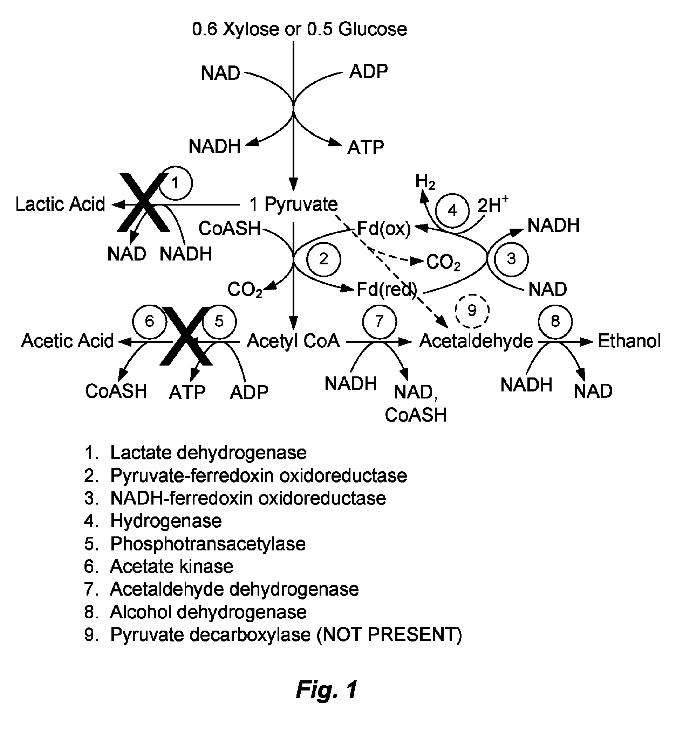

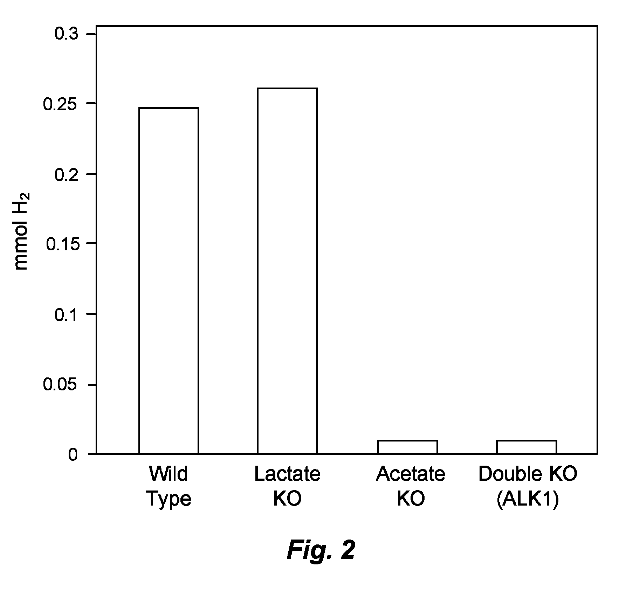

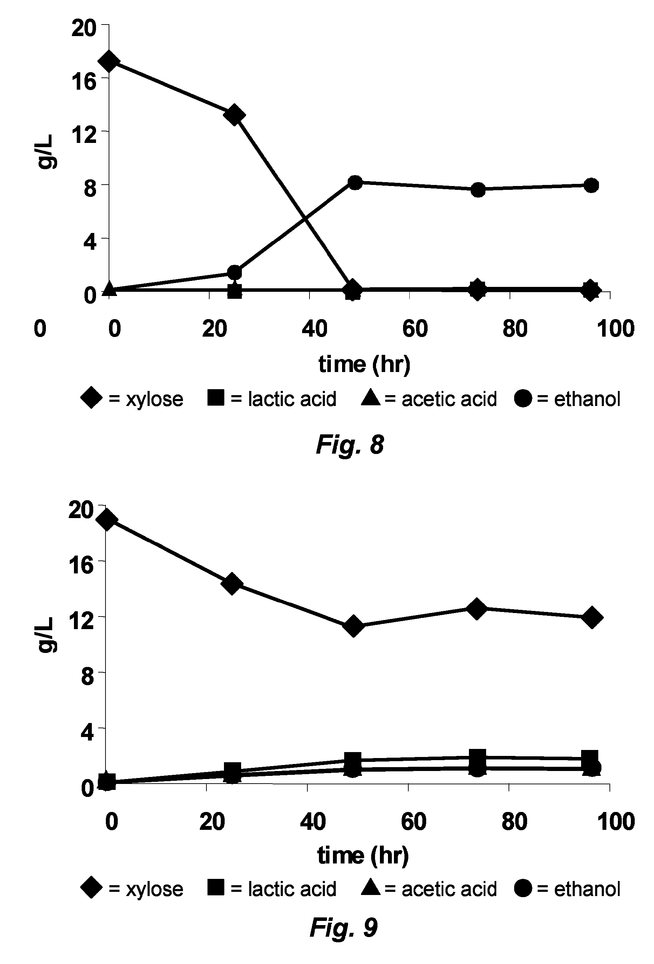

In contrast to known "homoethanol-fermenting" microorganisms, such as naturally-occurring *Saccharomyces cerevisiae* and *Zymomonas mobilis,* and recombinant strains of *Escherchia coli* and *Klebsiella oxytoca*, the presently disclosed organisms do not depend on conversion of pyruvate to acetaldehyde via the action of pyruvate decarboxylase (see Fig.1, 9). In fact, bacteria belonging to the *Thermoanaerobacter* genus do not express pyruvate decarboxylase in the native state. From the reactions of the glycolytic pathway shown in Fig. 1, it can be observed that pyruvate may be metabolized to acetyl-CoA, carbon dioxide, and reduced ferredoxin by the enzyme pyruvate-ferredoxin oxidoreductase 2. However, in order to produce ethanol as the only fermentation product, the electrons carried by reduced ferredoxin must all be transferred to NAD via NAD:ferredoxin oxidoreductase 3 to form NADH. NADH is subsequently oxidized back to NAD in the course of the two-step reduction of acetyl-CoA to ethanol by acetaldehyde dehydrogenase 7 and alcohol dehydrogenase 8. Evidence of the efficient utilization of NADH may be observed in Fig. 2 as a decrease in production of H₂ by both the acetate knockout organism, which is unable to express *ack* and *pta,* and the double knockout organism (ALK1), which is unable to express *ack*, *pta* and *ldh.* These organisms provide the first demonstration of stoichiometric electron transfer from reduced ferredoxin to NAD, and subsequently to ethanol.

The above-described pathway, which produces stoichiometric ethanol yields in organisms that do not possess the ability to express PDC, is in contrast to the pathway employed in all previously-described homoethanol-fermenting strains. Previously-described strains utilize endogenous pyruvate decarboxylase (PDC), or are engineered to express exogenous PDC. Since expression of PDC is rare in the microbial world, the ability to redirect electron flow by virtue of modifications to carbon flow has broad implications. For example, this approach could be used to produce high ethanol yields in strains other than *T*. *saccharolyticum* and/or to produce solvents other than ethanol. In particular, Gram-positive bacteria, such as members of the Thermoanaerober genus; *Clostridium thermocellum* and other thermophilic and mesophilic Clostridia_{;} thermophilic and mesophilic Bacillus species; Gram-negative bacteria, such as *Escherichia coli* and *Klebsiella oxytoca*; *Fibrobacter succinogenes* and other Fibrobacter species; *Thermoga neopolitana* and other Thermotoga species; and anaerobic fungi including Neocallimatix and Piromyces species lack the ability to express PDC, and may benefit from the disclosed instrumentalities.

It will be appreciated that lignocellulosic material that is saccharified to produce one or more of glucose, xylose, mannose, arabinose, galactose, fructose, cellobiose, sucrose, maltose, xylan, mannan and starch may be utilized by the disclosed organisms. In various embodiments, the lignocellulosic biomass comprises wood, corn stover, sawdust, bark, leaves, agricultural and forestry residues, grasses such as switchgrass, ruminant digestion products, municipal wastes, paper mill effluent, newspaper, cardboard, or combinations thereof.

### EXAMPLE 1

### PRODUCTION OF THE ALK1 STRAIN

### Materials and Methods

*Thermoanaerobacterium saccharolyticum* strain JW/SL-YS485 (DSM 8691) is a thermophilic, anaerobic bacteria isolated from the West Thumb Basin in Yellowstone National Park, Wyoming (Lui, S.Y; Gherardini, F.C.; Matuscheck, M.; Bahl, H.; Wiegel, J. "Cloning, sequencing, and expression of the gene encoding a large S-layer-associated endoxylanase from Thermoanaerobacterium sp strain JW/SL-YS485 in Escherichia coli" J. Bacteriol. 178: 1539-1547, 1996; Mai, V.; Wiegel, J. "Advances in development of a genetic system for Thermoanaerobacterium spp: Expression of genes encoding hydrolytic enzymes, development of a second shuttle vector, and integration of genes into the chromosome" Appl. Environ. Microbiol. 66: 4817-4821, 2000). It grows in a temperature range of 30-66°C and in a pH range of 3.85-6.5. It consumes a variety of biomass derived substrates including the monosaccharides glucose and xylose, the disaccharides cellobiose and sucrose, and the polysaccharides xylan and starch, but not cellulose. The organism produces ethanol as well as the organic acids lactic acid and acetic acid as primary fermentation products.

### Cloning and Sequencing

The lactate dehydrogenase (*L-ldh*)*,* phosphotransacetylase (*pta*)*,* and acetate kinase (*ack*) genes were identified and sequenced using standard techniques, as reported previously for *L-ldh* (Desai, 2004). Degenerate primers were made using the CODE-HOP algorithm (Rose,T.; Schultz, E.; Henikoff, J.; Pietrokovski, S.; McCallum, C.; Henikoff, S. "Consensus-degenerate hybrid oligonucleotide primers for amplification of distantly-related sequences" Nucleic Acids Research, 26(7):1628-1635, 1 Apr 1998) and PCR reactions were performed to obtain the DNA sequence between conserved regions. The gene fragments outside of the conserved regions were sequenced directly from genomic DNA using ThermoFidelase (Fidelity Systems, Gaithersburg, MD) enzyme with BigDye Terminator kit v3.1 (ABI, Foster City CA).

### Construction of Suicide Vectors

### Acetate kinase and phosphotransacetylase knockout vector, pSGD9

Standard cloning techniques were followed (Sambrook). The 6.2 kb suicide vector pSGD9 was based on pBLUESCRIPT II SK (+) (Stratagene) using a design approach similar to that reported earlier (Desai, 2004; Mai, 2000). Gene fragments of the *pta*/*ack* sequence,pta-up (∼1.2 kb) and *ack*-down (∼0.6 kb), were amplified from genomic DNA using primer pairs SEQ ID NOS: 1-2, and SEQ ID NOS: 3-4. PCR amplification was performed with pfu DNA polymerase and the fragments were extracted from a 1% electrophoresis gel. Fragments pta-up and ack-down were then A-tailed with Taq polymerase and cloned into TOPO pCR2.1 (Invitrogen, Carlsbad, CA). A 1.5 kb fragment containing the kanamycin marker was obtained from a *PstI*/*XbaI* digest of pIKM1 and subcloned into pBLUESCRIPT II SK (+). TOPO containing *pta*-up was digested with *XhoI*/*BsiHKAI* and subcloned into *XhoI*/*PstI* digested pBLUESCRIPT II SK (+), upstream of the previously subcloned kanamycin marker. TOPO containing *ack*-down was digested wi*th XbaI*/*SphI* and subcloned into pUC19 (Invitrogen). *XbaIlAflIII* fragment containing *ack*-down was digested and subcloned downstream of the kanamycin marker to obtain the final construct pSGD9.

### Lactate dehydrogenase knockout vector with erythromycin resistance, pCD8-Erm

The 5.5 kb suicide vector pSGD8-Erm was based on the plasmid pSGD8 as produced by Desai et al. 2004. In place of the *aph* kanamycin antibiotic marker, a fusion gene based on the *aph* promoter from the plasmid pIKM1 and the adenine methylase gene conferring erythromycin resistance from the plasmid pCTC1 (Klapatch, T.R.; Guerinot, M.L.; Lynd, L.R. "Electrotransformation of Clostridium thermosaccharolyticum" J. Ind. Microbiol. 16(6): 342-7, June 1996) were used for selection. PCR gene fragments were created using pfu polymerase (Stategene) and the primers SEQ ID NOS: 5-6 for the *aph* promoter and SEQ ID NOS: 7-8 for the adenine methylase open reading frame. Fragments were digested with *XbaI*/*BamH1* (*aph* fragment) and *BamHI*/*EcoRI* (adenine methylase) and ligated into the multiple cloning site of pIKM1. This fusion gene was then excised with *BseRIlEcoRI* and ligated into similarly digested pSGD8.

### Transformation of T. saccharolyticum

Transformation of *T. saccharolyticum* was performed interchangeably with two methods, the first as previously described (Mai, V.; Lorenz, W.; Weigel, J. "Transformation of Thermoanaerobacterium sp. strain JW/SL-YS485 with plasmid PIKM1 conferring kanamycin resistance" FEMS Microbiol. Lett. 148: 163-167,1997) and the second with several modifications following cell harvest and based on the method developed for *Clostridium thermocellum* (Tyurin, M. V.; Desai, S. G.; Lynd, L. R, "Electrotransformation of *Clostridium thermocellum"* Appl. Environ. Microbiol. **70**(2): 883-890,2004). Cells were grown overnight using pre-reduced medium DSMZ 122 in sterile disposable culture tubes inside an anaerobic chamber in an incubator maintained at 55°C. Thereafter, cells were sub-cultured with 4 µg/ml isonicotonic acid hydrazide (isoniaein), a cell wall weakening agent (Hermans, J.; Boschloo, J.G.; de Bont, J.A.M. "Transformation of M. aurum by electroporation: the use of glycine, lysozyme and isonicotinic acid hydrazide in enhancing transformation efficiency" FEMS Microbiol. Lett. 72, 221-224,1990), added to the medium after the initial lag phase. Exponential phase cells were harvested and washed with pre-reduced cold sterile 200mM cellobiose solution, and resuspended in the same solution and kept on ice. Extreme care was taken following the harvesting ofcells to keep them cold (approximately 4°C) at all times including the time during centrifugation.

Samples composed of 90 µl of the cell suspension and 2 to 6 µl of pSGD9 or pSOD8-Erm (1 to 3 µg) added just before pulse application, were placed into sterile 2 ml polypropylene microcentrifuge disposable tubes that served as electrotransformation cuvettes. A square-wave with pulse length set at 10ms was applied using a custom-built pulse generator/titanium electrode system. A voltage threshold corresponding to the formation of electropores in a cell sample was evaluated as a non-linear current change when pulse voltage was linearly increased in 200V increments. A particular voltage that provided the best ratio of transformation yield versus cell viability rate at a given DNA concentration was used, which in this particular case was 25kV/cm. Pulsed cells were initially diluted with 500 µl DSM 122 medium, held on ice for 10 minutes and then recovered at 55°C for 4-6 hrs. Following recovery, cells transformed with pSGD9 were mixed with 2% agar medium containing kanamycin at 75 µg/ml and poured onto petri plates and incubated in anaerobic jars for 4 days. Cells transformed with pSGD8-Erm were allowed to recover at 48°C for 4-6 hrs and were either plated in 2% agar medium at pH 6.0 containing erythromycin at 5 µg/ml or similar liquid media and incubated in anaerobic jars at 48°C for 6 days. Either of the transformed cell lines may be used without further manipulation. However, an organism where elimination of expression of all genes that confer the ability to produce organic acids was obtained by performing a second (sequential) transformation. The second transformation was carried out as described above with the primary transformant substituted for the non-transformed cell suspension. The secondary transformant, ALK1, was grown on medium containing both kanamycin and erythromycin.

### Sequencing of Knockout Regions

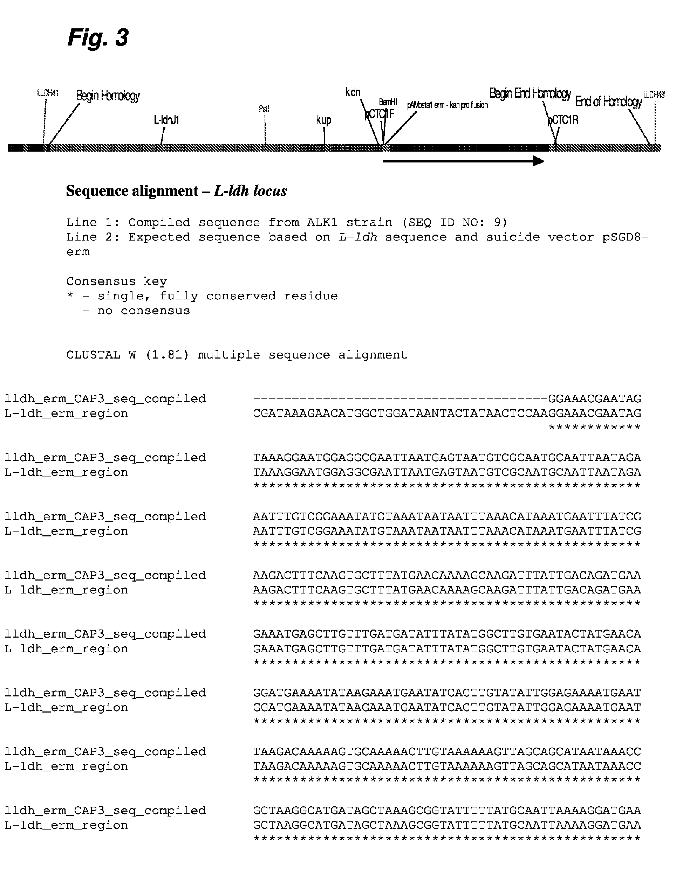

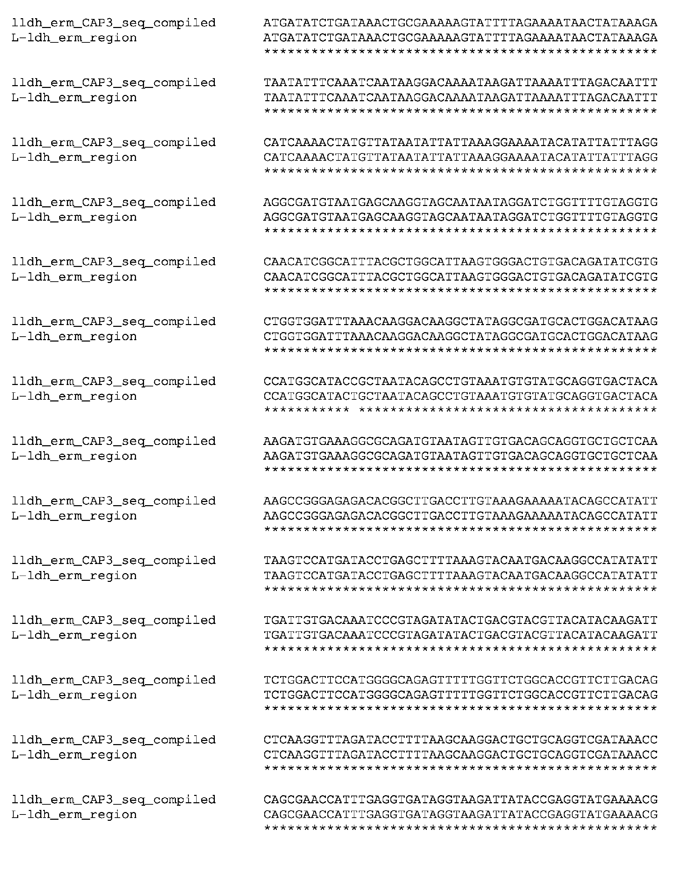

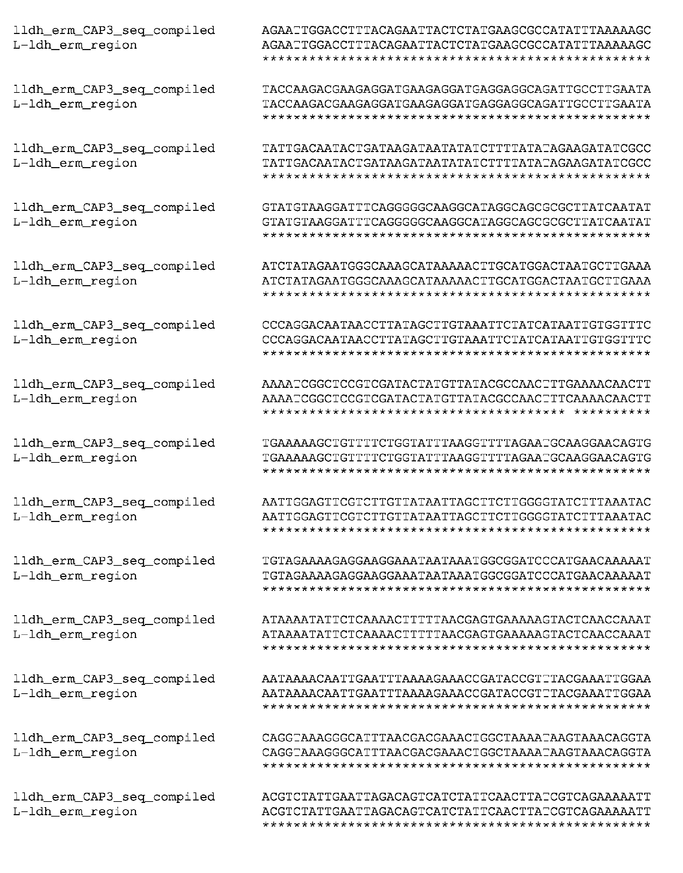

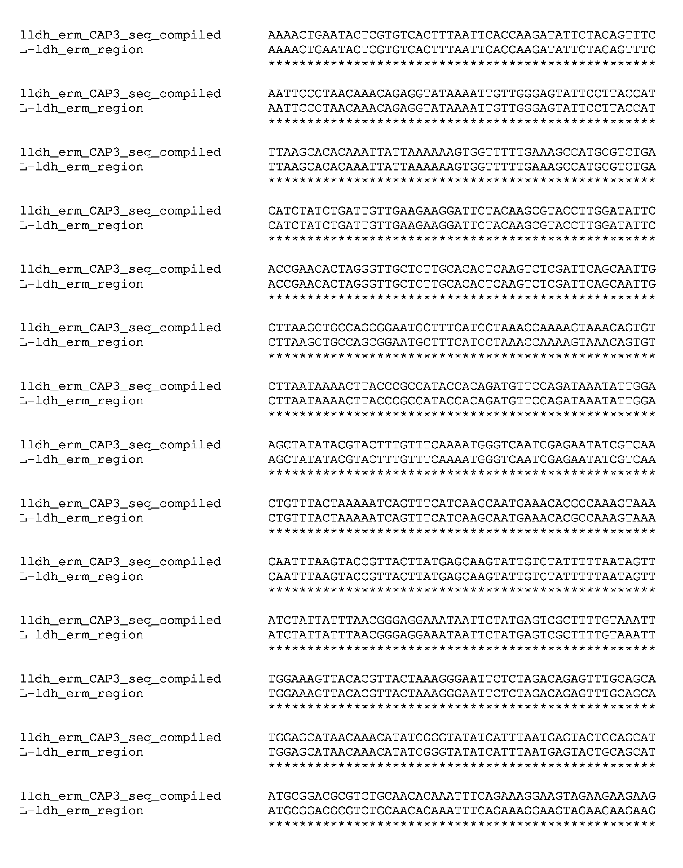

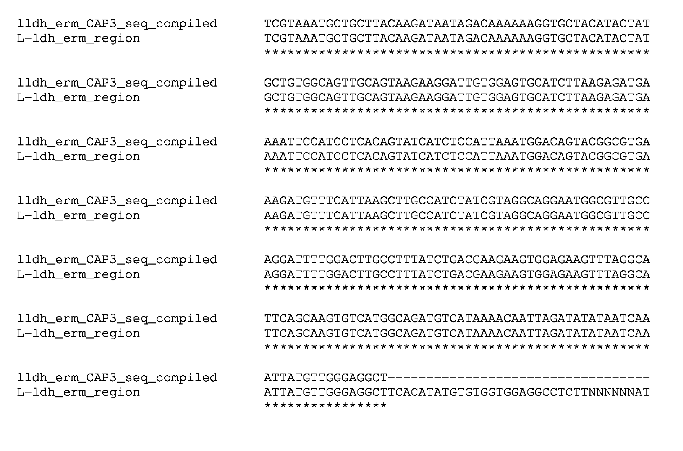

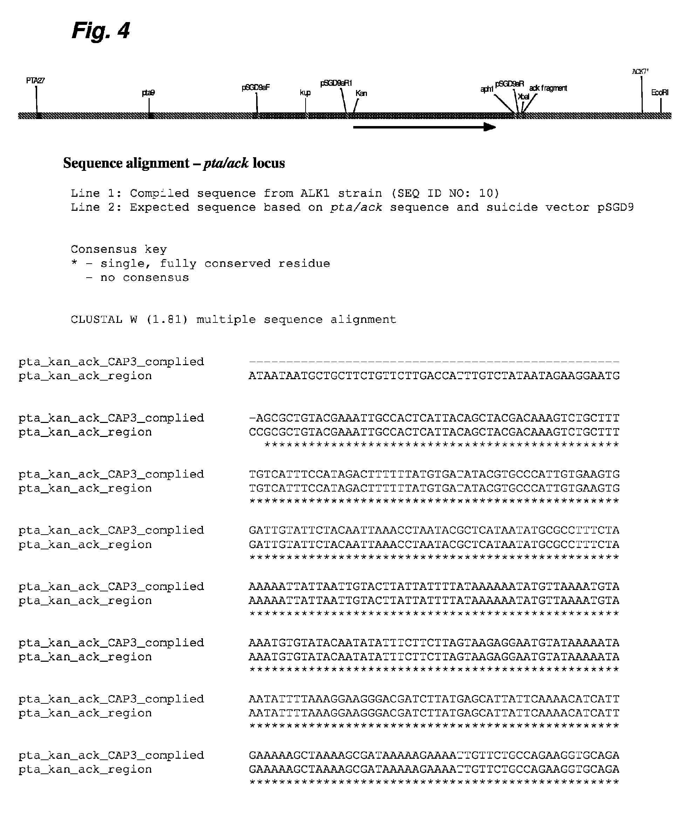

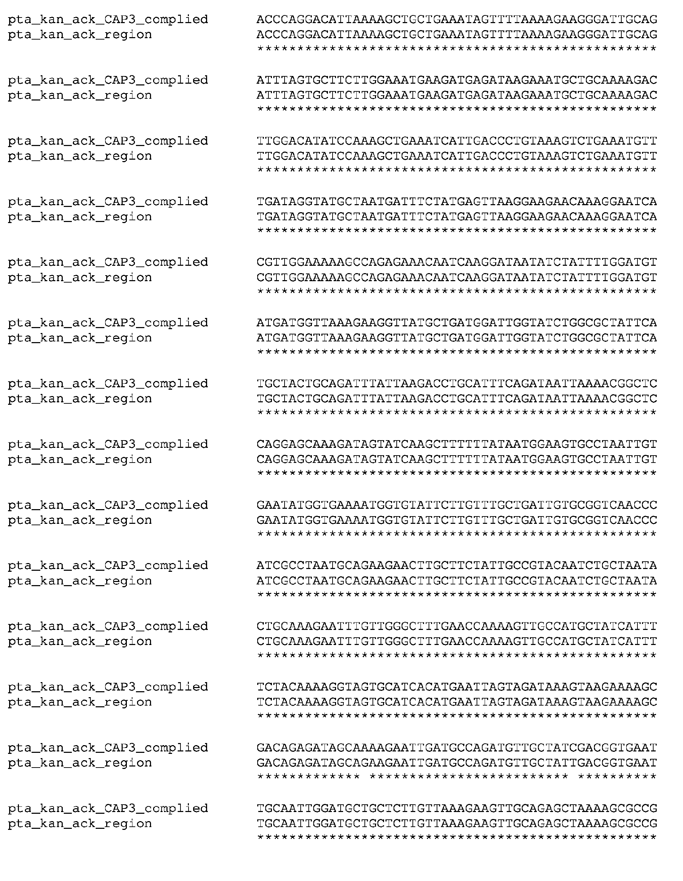

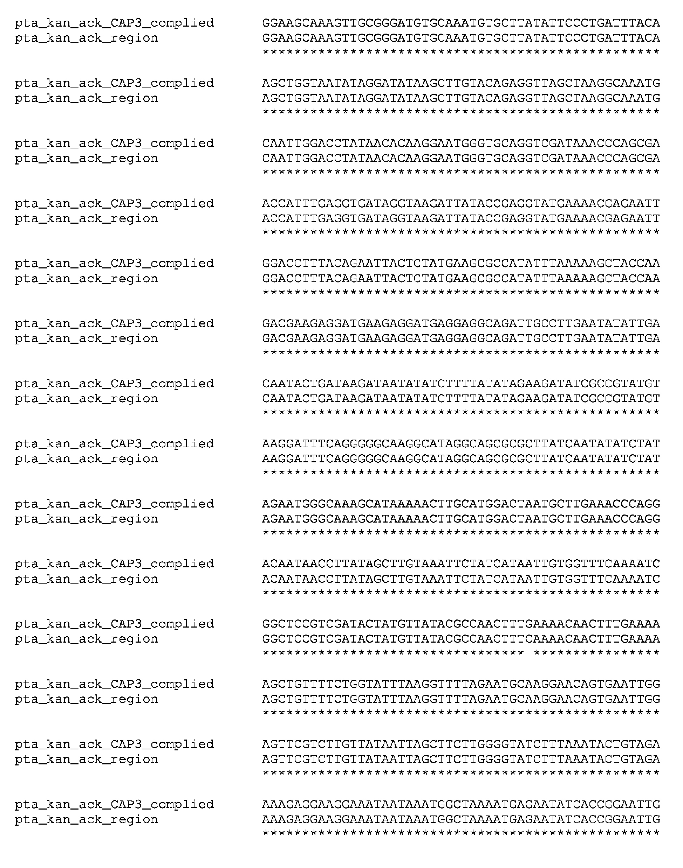

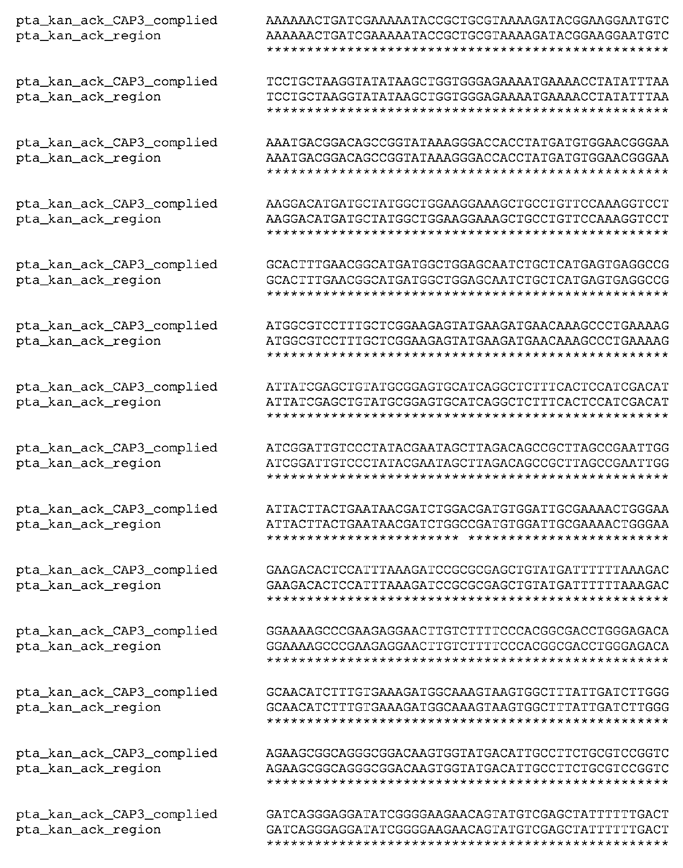

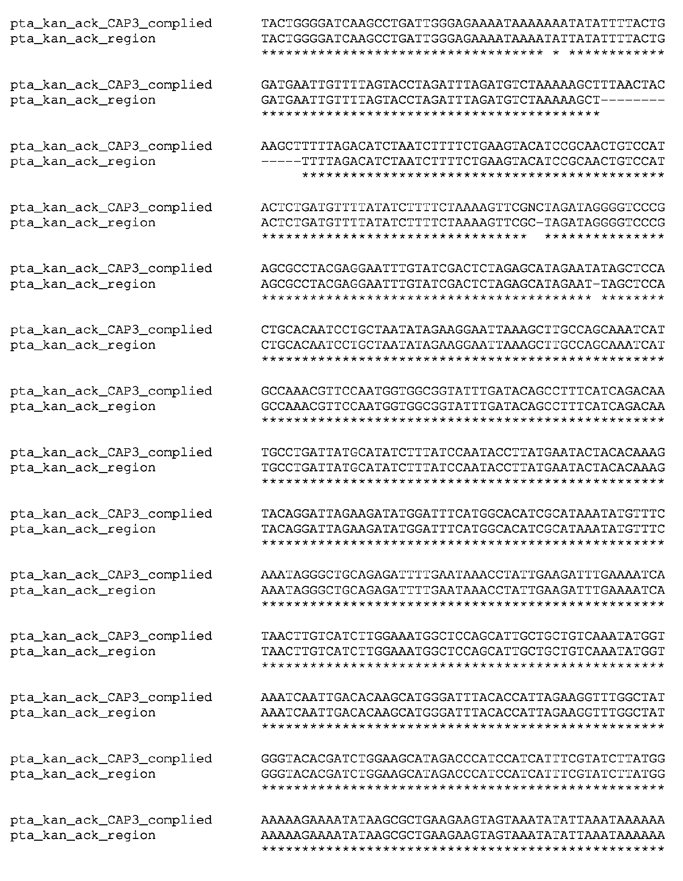

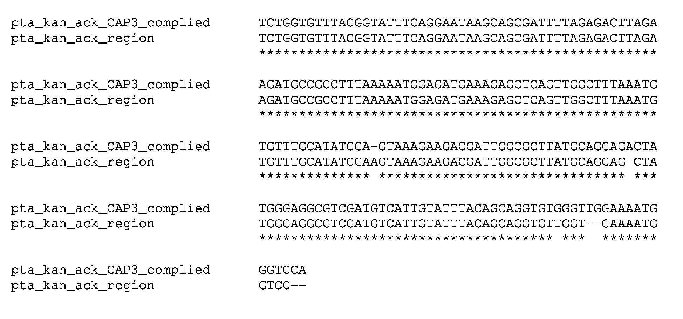

Sequencing of the site directed knockout regions was done by PCR from genomic DNA using Taq polymerase (New England Biolabs) and primers outside the regions of homologous overlap between the genome arid the suicide vectors. Primers inside the PCR products were used for sequencing with the BigDye Terminator kit v3.1 (ABI, Foster City, CA). Regions were arranged using the CAP3 software program (Huang, X. "An improved sequence assembly program" Genomics 33: 21-31, 1996) and compared to the expected DNA sequence using the CLUSTALW algorithm (Higgins, D. G.; Bleasby, A. J.; Fuchs, R. "CLUSTAL V: improved software for multiple sequence alignment" Computer Applications in the Biosciences (CABIOS), 8(2): 189-191, 1992). A high degree of homology (percent identity) existed between the experimentally compiled sequence and the expected sequence based on the known wild-type and suicide vector sequences (Figs. 3 and 4).

"Identity" refers to a comparison between pairs of nucleic acid or amino acid molecules. Methods for determining sequence identity are known. See, for example, computer programs commonly employed for this purpose, such as the Gap program (Wisconsin Sequence Analysis Package, Version 8 for Unix, Genetics Computer Group, University Research Park, Madison Wisconsin), that uses the algorithm of Smith and Waterman, 1981, Adv, Appl. Math. 2: 482-489.

### Verification of Mutant Strain

Genomic DNA from the mutant strain *Thermoanaerobacterium saccharolyticum* JW/SL-YS485 ALK1 ("ALK1") showed the expected site-directed homologous recombination in the *L-ldh* and pta/ack loci through DNA sequencing. Both integration events were double integrations, which is a more genetically stable genotype.

### EXAMPLE 2

### COMPARATIVE DATA SHOWING PRODUCTION OF ETHANOL BY ALK1 AND WILD-TYPE T. SACCHAROLYTICUM

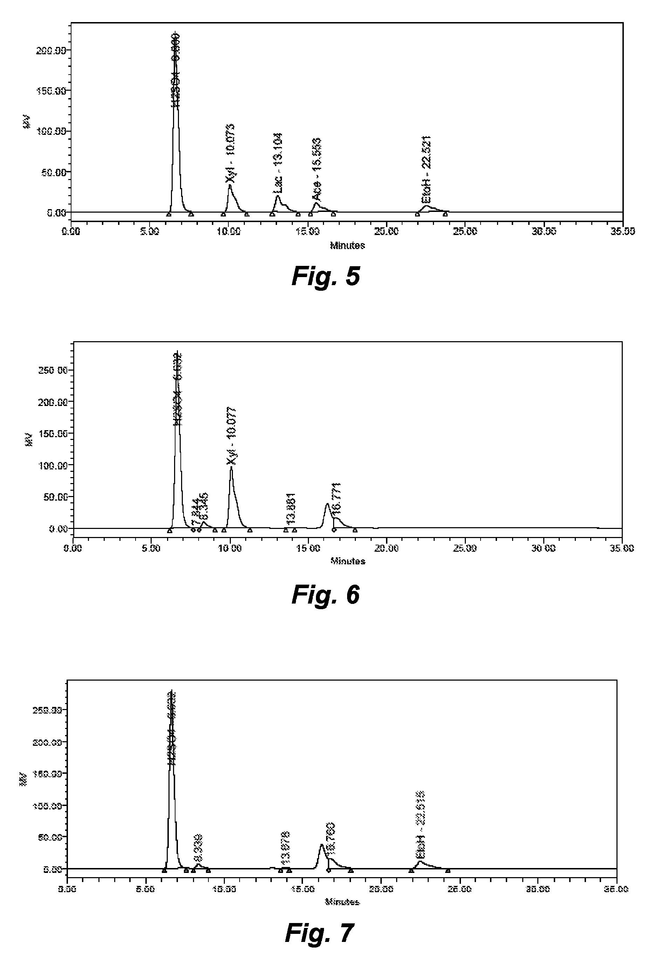

*T. saccharolyticum* was grown in partially defined MTC media containing 2.5 g/L Yeast Extract (Zhang, Y.; Lynd, L. R. "Quantification of cell and cellulase mass concentrations during anaerobic cellulose fermentation: development of an enzyme-linked immunosorbent assay-based method with application to Clostridium thermocellum batch cultures" Anal. Chem. 75: 219-222, 2003). Glucose, xylose, acetate, lactate and ethanol were analyzed by HPLC on an Aminex 87H column (BioRad Laboratories, Hercules, CA) at 55°C. The mobile phase consisted of 5mM sulfuric acid at a flow rate of 0.7ml/min. Detection was via refractive index using a Waters 410 refractometer (Milford, MA). The minimum detection level for acetate was 1.0 mM. A standard trace containing 5 g/L xylose, 5 g/L lactic acid, 5 g/L acetic acid and 5 g/L ethanol is shown in Fig. 5.

Strain ALK1 produced only ethanol with up to 17 g/L xylose, or with 5 g/L xylose and 5 g/L glucose, with no organic acids or other products detected by HPLC. Fig. 6 shows the ALK1 strain fermentation at time 0 hours and Fig. 7 shows the same fermentation at 72 hours. Time course fermentation plots of strain ALK1 and wild-type on xylose media buffered with 8 g/L MES at an initial pH of 6.0, 55°C and 100 rpm show that strain ALK1 is able to convert over 99% of xylose to ethanol (Fig. 8), while the wild-type under similar conditions becomes pH limited due to organic acid production and is unable to consume all the xylose present (Fig. 9). The wild-type organism yielded 0.15 mM ethanol, while ALK1 yielded 0.46 mM ethanol.

### EXAMPLE 3

### EVOLUTION OF ALK1

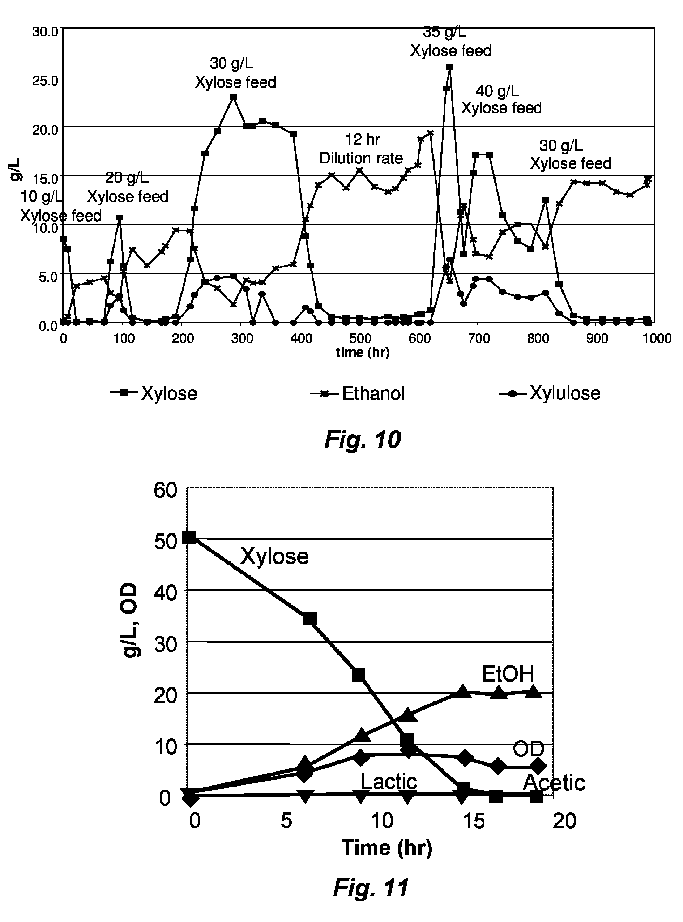

As shown in FIG. 10, a continuous culture in which feed substrate concentration was increased over time was utilized to challenge ALK1. Fig. 10 shows xylose, xylulose and ethanol concentrations during the continuous culture. After more than 1000 hours of exposure to this stress-evolution cycle, an improved strain, ALK2, was isolated from the fermentation broth. ALK2 was able to initiate growth at 50 g/L xylose in batch culture. Fig. 11 shows xylose, organic acid, optical density (OD) and ethanol concentrations during fermentation by strain ALK2.

### EXAMPLE 4

### THERMOPHILIC SIMULTANEOUS SACCHARIFICATION AND FERMENTATION

Some thermotolerant yeast strains have been tested for ethanol production via SSF at temperatures of 40-45°C, with reduced yield above these temperatures (Banat, I. M.; Nigam, P.; Singh, D.; Marchant, R.; McHale, A.P. "Review: Ethanol production at elevated temperatures and alcohol concentrations: Part I - Yeasts in general." World Journal of Microbiology and Biotechnology 14: 809-821,1998). In addition, Patel *et al.* have demonstrated SSF at 50°C for the production of lactic acid using a *Bacillus* strain (Patel, M. A.; Ou, M. S.; Ingram, L. O.; Shanmugam, K. T. "Simultaneous saccharification and co-fermentation of crystalline cellulose and sugar cane bagasse hemicellulose hydfolysate to lactate by a thermotolerant acidophilic Bacillus sp." Biotechnol. Prog. 21: 1453-1460, 2005). Thermophilic SSF has not, however, been previously demonstrated at 50°C for the production of ethanol with reduced enzyme requirements.

As discussed above, thermophilie organisms transformed according to the present disclosure have the potential to contribute significant savings in lignocellulosic biomass to ethanol conversion due to their growth conditions, which are substantially optimal for cellulase activity in SSF processes. For example, ALK1 and ALK2 are anaerobic thermophiles that can grow at 50°C and pH 5.0, while optimal cellulase activity parameters include a pH between 4-5 and temperature between 40-50°C.

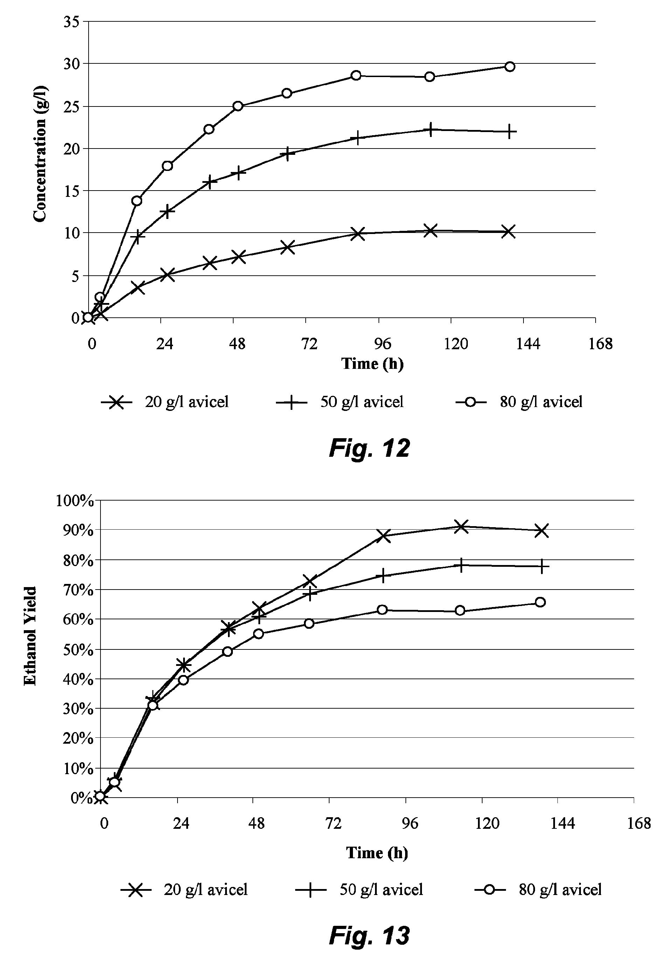

In a thermophilic simultaneous saccharification and fermentation (tSSF) reaction performed on a 1.5 L scale in a batch reactor at 50°C, ALK2 was used in conjunction with 4 FPU/g *T*. *reesei* cellulase (Genencor, Palo Alto, CA) to produce ethanol from the solid substrate Avicel (20 g/L, 50 g/L and 80 g/L). There were no soluble sugars measured after sixteen hours of fermentation and less than 0.5 g/L lactic acid were produced in any of the trials. Fig. 12 shows that 30 g/L ethanol were produced in 140 hours when the tSSF reaction was performed on 80 g/l Avicel, with 25 of the 30g/L ethanol produced in the first 50 hours. Fig. 13 shows ethanol yields for the reactions illustrated in Fig. 12. Yields were calculated based upon the theoretical maximum of 0.51 g ethanol per gram glucose equivalent. At 20 g/L Avicel, about 90% conversion was achieved in 140 hours.

It will be appreciated that *T*. *saccharolyticum* can ferment both pentose and hexose sugars. The disclosed organisms can therefore be used in Simultaneous Saccharification and Co-Fermentation (SSCF) reactions, where an enzyme that converts hemicellulose into pentose sugars (e.g., xylase) may be utilized in combination with cellulase.

### Deposit of ALK1

ALK1 has been deposited with the American Type Culture Collection, Manassas, VA 20110-2209. The deposit was made on November 1, 2005 and received Patent Deposit Designation Number PTA-7206. This deposit was made in compliance with the Budapest Treaty requirements that the duration of the deposit should be for thirty (30) years from the date of deposit or for five (5) years after the last request for the deposit at the depository or for the enforceable life of a U.S. Patent that matures from this application, whichever is longer. ALK1 will be replenished should it become non-viable at the depository.

The description of the specific embodiments reveals general concepts that others can modify and/or adapt for various applications or uses that do not depart from the general concepts. Therefore, such adaptations and modifications should and are intended to be comprehended within the meaning and range of equivalents of the disclosed embodiments. It is to be understood that the phraseology or terminology employed herein is for the purpose of description and not limitation.

The foregoing examples may be suitably modified for use upon any Gram-positive bacterium, and especially members of the *Thermoanaerobacter* genus including *Thermoanaerobacterium thermosulfurigenes, Thermoanaerobacterium aotearoense, Thermoanaerobacterium polysaccharolyticum, Thermoanaerobacterium zeae, Thermoanaerobacterium thermosaccharolyticum*, and *Thermoanaerobacterium xylanolyticum.*

All references mentioned in this application are incorporated by reference to the same extent as though fully replicated herein,

## Claims

1. A method for producing ethanol, said method comprising:
providing within a reaction vessel, a reaction mixture comprising lignocellulosic substrate, cellulase and a fermentation agent, the fermentation agent comprising a Gram-positive bacterium that has been transformed to eliminate expression of at least one gene that confers upon the Gram-positive bacterium, in a native state, an ability to produce acetic acid as a fermentation product,
wherein the reaction mixture is reacted under suitable conditions for a period of time sufficient to allow saccharification and fermentation of the lignocellulosic substrate.

2. The method of claim 1, wherein the suitable conditions comprise a temperature of at least 50°C.

3. The method of claim 1, wherein the Gram-positive bacterium is a member of the *Thermoanaerobacter* genus.

4. The method of claim 1, wherein the Gram-positive bacterium is a *Thermoanaerobacterium saccharolyticum*.

5. The method of claim 4, wherein the bacterium is *Thermoanaerobacterium saccharolyticum* ALK1 (JW/SL-YS485 ALK1).

6. The method of claim 4, wherein the bacterium is *Thermoanaerobacterium saccharolyticum* ALK2.

7. The method of claim 1, wherein the at least one gene encodes an acetate kinase.

8. The method of claim 1, wherein the at least one gene encodes a phosphotransacetylase.

9. The method of claim 1, wherein the at least one gene includes a plurality of genes.

10. The method of claim 9, wherein the plurality of genes encode acetate kinase and phosphotransacetylase.

11. The method of claim 10, further comprising transforming the Gram-positive bacterium to eliminate expression of one or more genes that confer upon the Gram-positive bacterium the ability to produce lactic acid as a fermentation product.

12. The method of claim 11, wherein the at least one of said one or more genes encodes a lactate dehydrogenase.

13. The method of claim 1, wherein at least one gene that confers upon the Gram-positive bacterium the ability to produce lactic acid as a fermentation product has been eliminated.

14. The method of claim 13, wherein the at least one gene encodes a lactate dehydrogenase.

15. A method for producing ethanol, said method comprising:
(a) transforming a Gram-positive bacterium to produce a transformed bacterial host by eliminating expression of at least one gene; wherein said at least one gene is selected from the group consisting of phosphotransacetylase (pta) gene, acetate kinase (ack) gene, and combination thereof, said Gram-positive bacterium belonging to the *Thermoanaerobacterium* genus; and
(b) culturing the transformed bacterial host in a medium containing a substrate under suitable conditions for a period of time sufficient to allow saccharification and fermentation of the substrate, said substrate being selected from the group consisting of glucose, xylose, mannose, arabinose, galactose, fructose, cellobiose, sucrose, maltose, xylan, mannan, starch, and combinations thereof.

16. The method according to claim 15, wherein said Gram-positive bacterium is *Thermoanaerobacterium saccharolyticum.*

17. The method according to claim 16, wherein said Gram-positive bacterium is *Thermoanaerobacterium saccharolyticum* strain ALK1 having a Patent Deposit Designation No. PTA-7206.

18. The method according to claim 16, wherein said Gram-positive bacterium is *Thermoanaerobacterium saccharolyticum* strain ALK2.

19. A biologically pure culture of a microorganism designated ALK2.


SEQUENCE LISTING

<110>  The Trustees of Dartmouth College
       Lynd, Lee R.
       Shaw, Arthur Josephus
       Desai, Sunil G.
       Tyurin, Mikhail V.
       Podkaminer, Kara
       Bardsley, John
       Hogsett, David

<120>  Thermophilic Organisms for Conversion of Lignocellulosic Biomass
       to Ethanol

<130>  466905

<140>  Not Yet Assigned
<141>  2007-05-01

<150>  US 60/731,674
<151>  2005-10-31

<150>  US 60/796,380
<151>  2006-05-01

<150>  PCT/US06/042442
<151>  2006-10-31

<160>  10

<170>  PatentIn version 3.2

<210>  1
<211>  34
<212>  DNA
<213>  Artificial Sequence

<220>
<223>  pta primer

<400>  1
acatgcatgc ccatttgtct ataatagaag gaag                                    34


<210>  2
<211>  24
<212>  DNA
<213>  Artificial Sequence

<220>
<223>  pta primer

<400>  2
cgtcaacaat atctctatag ctgc                                               24


<210>  3
<211>  29
<212>  DNA
<213>  Artificial Sequence

<220>
<223>  ack primer

<400>  3
gctctagagc atagaattag ctccactgc                                          29


<210>  4
<211>  34
<212>  DNA
<213>  Artificial Sequence

<220>
<223>  ack primer

<400>  4
acatgcatgc cgacgcctcc catagctgct gcat                                    34


<210>  5
<211>  34
<212>  DNA
<213>  Artificial Sequence

<220>
<223>  aph primer

<400>  5
tggatccgcc atttattatt tccttcctct tttc                                    34


<210>  6
<211>  27
<212>  DNA
<213>  Artificial Sequence

<220>
<223>  aph primer

<400>  6
ttctagatgg ctgcaggtcg ataaacc                                            27


<210>  7
<211>  34
<212>  DNA
<213>  Artificial Sequence

<220>
<223>  adenine methylase primer

<400>  7
gcggatccca tgaacaaaaa tataaaatat tctc                                    34


<210>  8
<211>  31
<212>  DNA
<213>  Artificial Sequence

<220>
<223>  adenine methylase primer

<400>  8
gcgaattccc tttagtaacg tgtaactttc c                                    31


<210>  9
<211>  2778
<212>  DNA
<213>  Artificial Sequence

<220>
<223>  L-ldh locus of Thermoanaerobacterium saccharolyticum knockout

<400>  9
ggaaacgaat agtaaaggaa tggaggcgaa ttaatgagta atgtcgcaat gcaattaata      60

gaaatttgtc ggaaatatgt aaataataat ttaaacataa atgaatttat cgaagacttt     120

caagtgcttt atgaacaaaa gcaagattta ttgacagatg aagaaatgag cttgtttgat     180

gatatttata tggcttgtga atactatgaa caggatgaaa atataagaaa tgaatatcac     240

ttgtatattg gagaaaatga attaagacaa aaagtgcaaa aacttgtaaa aaagttagca     300

gcataataaa ccgctaaggc atgatagcta aagcggtatt tttatgcaat taaaaggatg     360

aaatgatatc tgataaactg cgaaaaagta ttttagaaaa taactataaa gataatattt     420

caaatcaata aggacaaaat aagattaaaa tttagacaat ttcatcaaaa ctatgttata     480

atattattaa aggaaaatac atattattta ggaggcgatg taatgagcaa ggtagcaata     540

ataggatctg gttttgtagg tgcaacatcg gcatttacgc tggcattaag tgggactgtg     600

acagatatcg tgctggtgga tttaaacaag gacaaggcta taggcgatgc actggacata     660

agccatggca taccgctaat acagcctgta aatgtgtatg caggtgacta caaagatgtg     720

aaaggcgcag atgtaatagt tgtgacagca ggtgctgctc aaaagccggg agagacacgg     780

cttgaccttg taaagaaaaa tacagccata tttaagtcca tgatacctga gcttttaaag     840

tacaatgaca aggccatata tttgattgtg acaaatcccg tagatatact gacgtacgtt     900

acatacaaga tttctggact tccatggggc agagtttttg gttctggcac cgttcttgac     960

agctcaaggt ttagatacct tttaagcaag gactgctgca ggtcgataaa cccagcgaac    1020

catttgaggt gataggtaag attataccga ggtatgaaaa cgagaattgg acctttacag    1080

aattactcta tgaagcgcca tatttaaaaa gctaccaaga cgaagaggat gaagaggatg    1140

aggaggcaga ttgccttgaa tatattgaca atactgataa gataatatat cttttatata    1200

gaagatatcg ccgtatgtaa ggatttcagg gggcaaggca taggcagcgc gcttatcaat    1260

atatctatag aatgggcaaa gcataaaaac ttgcatggac taatgcttga aacccaggac    1320

aataacctta tagcttgtaa attctatcat aattgtggtt tcaaaatcgg ctccgtcgat 1380

actatgttat acgccaactt tgaaaacaac tttgaaaaag ctgttttctg gtatttaagg 1440

ttttagaatg caaggaacag tgaattggag ttcgtcttgt tataattagc ttcttggggt 1500

atctttaaat actgtagaaa agaggaagga aataataaat ggcggatccc atgaacaaaa 1560

atataaaata ttctcaaaac tttttaacga gtgaaaaagt actcaaccaa ataataaaac 1620

aattgaattt aaaagaaacc gataccgttt acgaaattgg aacaggtaaa gggcatttaa 1680

cgacgaaact ggctaaaata agtaaacagg taacgtctat tgaattagac agtcatctat 1740

tcaacttatc gtcagaaaaa ttaaaactga atactcgtgt cactttaatt caccaagata 1800

ttctacagtt tcaattccct aacaaacaga ggtataaaat tgttgggagt attccttacc 1860

atttaagcac acaaattatt aaaaaagtgg tttttgaaag ccatgcgtct gacatctatc 1920

tgattgttga agaaggattc tacaagcgta ccttggatat tcaccgaaca ctagggttgc 1980

tcttgcacac tcaagtctcg attcagcaat tgcttaagct gccagcggaa tgctttcatc 2040

ctaaaccaaa agtaaacagt gtcttaataa aacttacccg ccataccaca gatgttccag 2100

ataaatattg gaagctatat acgtactttg tttcaaaatg ggtcaatcga gaatatcgtc 2160

aactgtttac taaaaatcag tttcatcaag caatgaaaca cgccaaagta aacaatttaa 2220

gtaccgttac ttatgagcaa gtattgtcta tttttaatag ttatctatta tttaacggga 2280

ggaaataatt ctatgagtcg cttttgtaaa tttggaaagt tacacgttac taaagggaat 2340

tctctagaca gagtttgcag catggagcat aacaaacata tcgggtatat catttaatga 2400

gtactgcagc atatgcggac gcgtctgcaa cacaaatttc agaaaggaag tagaagaaga 2460

agtcgtaaat gctgcttaca agataataga caaaaaaggt gctacatact atgctgtggc 2520

agttgcagta agaaggattg tggagtgcat cttaagagat gaaaattcca tcctcacagt 2580

atcatctcca ttaaatggac agtacggcgt gaaagatgtt tcattaagct tgccatctat 2640

cgtaggcagg aatggcgttg ccaggatttt ggacttgcct ttatctgacg aagaagtgga 2700

gaagtttagg cattcagcaa gtgtcatggc agatgtcata aaacaattag atatataatc 2760

aaattatgtt gggaggct 2778

<210> 10
<211> 3355
<212> DNA
<213> Artificial Sequence

<220>
<223> pta/ack locus of Thermoanaerobacterium saccharolyticum knockout

<220>
<221>  misc_feature
<222>  (2632)..(2634)
<223>  n is a, c, g or t

<220>
<221>  misc_feature
<222>  (3262)..(3265)
<223>  n is a, c, g, or t

<400>  10
agcgctgtac gaaattgcca ctcattacag ctacgacaaa gtctgctttt gtcatttcca      60

tagacttttt tatgtgatat acgtgcccat tgtgaagtgg attgtattct acaattaaac     120

ctaatacgct cataatatgc gcctttctaa aaaattatta attgtactta ttattttata     180

aaaaatatgt taaaatgtaa aatgtgtata caatatattt cttcttagta agaggaatgt     240

ataaaaataa atattttaaa ggaagggacg atcttatgag cattattcaa aacatcattg     300

aaaaagctaa aagcgataaa aagaaaattg ttctgccaga aggtgcagaa cccaggacat     360

taaaagctgc tgaaatagtt ttaaaagaag ggattgcaga tttagtgctt cttggaaatg     420

aagatgagat aagaaatgct gcaaaagact tggacatatc caaagctgaa atcattgacc     480

ctgtaaagtc tgaaatgttt gataggtatg ctaatgattt ctatgagtta aggaagaaca     540

aaggaatcac gttggaaaaa gccagagaaa caatcaagga taatatctat tttggatgta     600

tgatggttaa agaaggttat gctgatggat tggtatctgg cgctattcat gctactgcag     660

atttattaag acctgcattt cagataatta aaacggctcc aggagcaaag atagtatcaa     720

gcttttttat aatggaagtg cctaattgtg aatatggtga aaatggtgta ttcttgtttg     780

ctgattgtgc ggtcaaccca tcgcctaatg cagaagaact tgcttctatt gccgtacaat     840

ctgctaatac tgcaaagaat ttgttgggct ttgaaccaaa agttgccatg ctatcatttt     900

ctacaaaagg tagtgcatca catgaattag tagataaagt aagaaaagcg acagagatag     960

caaaagaatt gatgccagat gttgctatcg acggtgaatt gcaattggat gctgctcttg    1020

ttaaagaagt tgcagagcta aaagcgccgg gaagcaaagt tgcgggatgt gcaaatgtgc    1080

ttatattccc tgatttacaa gctggtaata taggatataa gcttgtacag aggttagcta    1140

aggcaaatgc aattggacct ataacacaag gaatgggtgc aggtcgataa acccagcgaa    1200

ccatttgagg tgataggtaa gattataccg aggtatgaaa acgagaattg gacctttaca    1260

gaattactct atgaagcgcc atatttaaaa agctaccaag acgaagagga tgaagaggat    1320

gaggaggcag attgccttga atatattgac aatactgata agataatata tcttttatat    1380

agaagatatc gccgtatgta aggatttcag ggggcaaggc ataggcagcg cgcttatcaa    1440

tatatctata gaatgggcaa agcataaaaa cttgcatgga ctaatgcttg aaacccagga 1500
caataacctt atagcttgta aattctatca taattgtggt ttcaaaatcg gctccgtcga 1560
tactatgtta tacgccaact ttgaaaacaa ctttgaaaaa gctgttttct ggtatttaag 1620
gttttagaat gcaaggaaca gtgaattgga gttcgtcttg ttataattag cttcttgggg 1680
tatctttaaa tactgtagaa aagaggaagg aaataataaa tggctaaaat gagaatatca 1740
ccggaattga aaaaactgat cgaaaaatac cgctgcgtaa aagatacgga aggaatgtct 1800
cctgctaagg tatataagct ggtgggagaa aatgaaaacc tatatttaaa aatgacggac 1860
agccggtata aagggaccac ctatgatgtg gaacgggaaa aggacatgat gctatggctg 1920
gaaggaaagc tgcctgttcc aaaggtcctg cactttgaac ggcatgatgg ctggagcaat 1980
ctgctcatga gtgaggccga tggcgtcctt tgctcggaag agtatgaaga tgaacaaagc 2040
cctgaaaaga ttatcgagct gtatgcggag tgcatcaggc tctttcactc catcgacata 2100
tcggattgtc cctatacgaa tagcttagac agccgcttag ccgaattgga ttacttactg 2160
aataacgatc tggacgatgt ggattgcgaa aactgggaag aagacactcc atttaaagat 2220
ccgcgcgagc tgtatgattt tttaaagacg gaaaagcccg aagaggaact tgtcttttcc 2280
cacggcgacc tgggagacag caacatcttt gtgaaagatg gcaaagtaag tggctttatt 2340
gatcttggga gaagcggcag ggcggacaag tggtatgaca ttgccttctg cgtccggtcg 2400
atcagggagg atatcgggga agaacagtat gtcgagctat tttttgactt actggggatc 2460
aagcctgatt gggagaaaat aaaaaaatat attttactgg atgaattgtt ttagtaccta 2520
gatttagatg tctaaaaagc tttaactaca agctttttag acatctaatc ttttctgaag 2580
tacatccgca actgtccata ctctgatgtt ttatatcttt tctaaaagtt cgnctagata 2640
ggggtcccga gcgcctacga ggaatttgta tcgactctag agcatagaat atagctccac 2700
tgcacaatcc tgctaatata gaaggaatta aagcttgcca gcaaatcatg ccaaacgttc 2760
caatggtggc ggtatttgat acagcctttc atcagacaat gcctgattat gcatatcttt 2820
atccaatacc ttatgaatac tacacaaagt acaggattag aagatatgga tttcatggca 2880
catcgcataa atatgtttca aatagggctg cagagatttt gaataaacct attgaagatt 2940
tgaaaatcat aacttgtcat cttggaaatg gctccagcat tgctgctgtc aaatatggta 3000
aatcaattga cacaagcatg ggatttacac cattagaagg tttggctatg ggtacacgat 3060
ctggaagcat agacccatcc atcatttcgt atcttatgga aaaagaaaat ataagcgctg 3120
aagaagtagt aaatatatta aataaaaaat ctggtgttta cggtatttca ggaataagca 3180
gcgattttag agacttagaa gatgccgcct ttaaaaatgg agatgaaaga gctcagttgg 3240

ctttaaatgt gtttgcatat cgangtaaag aagacgattg gcgcttatgc agcagactat 3300

gggaggcgtc gatgtcattg tatttacagc aggtgtgggt tggaaaatgg gtcca 3355